# EUROPÄISCHE PATENTANMELDUNG

(11) **EP 1 228 749 A2**
(43) Veröffentlichungstag der Anmeldung: **07.08.2002**
(21) Anmeldenummer: 02000015.4
(22) Anmeldetag: 03.01.2002
(51) Int. Cl.: A61K 7/06

(54) **Elektrisch leitfähige Polymere in kosmetischen Mitteln**

(30) Priorität: 06.02.2001 DE 10105139
(71) Anmelder: Wella Aktiengesellschaft, 64274 Darmstadt (DE)
(72) Erfinder: Sendelbach, Gerhard, 64297 Darmstadt (DE); Krause, Thomas, 64297 Darmstadt (DE); Duchscherer, Anja, 65589 Hadamar (DE)

(57) **Zusammenfassung**

Es werden kosmetische Mittel beschrieben, welche aufgrund eines Gehaltes an elektrisch leitfähigen Polymeren in der Lage sind, bereits in sehr geringen Einsatzkonzentrationen den fly-away-Effekt von Haaren zu verringern.

## Beschreibung

Gegenstand der Erfindung ist ein kosmetisches Mittel mit einem Gehalt an einem elektrisch leitfähigen Polymer. Das Mittel ist insbesondere als Haarpflegemittel in Form eines Shampoos oder einer Haarkur anwendbar und verringert den fly-away-Effekt von behandelten Haaren.

Frisch gewaschenes Haar, insbesondere dünnes Haar neigt nach dem Trocknen dazu, durch statische Aufladung vom Kopf abzustehen und durch diesen sogenannten fly-away-Effekt das Frisurenbild zu stören. Konventionelle Lösungen für dieses Problem sind entweder die Verwendung von Stylingmitteln wie Haarsprays, Haarfestiger oder Schäume, die aber den Nachteil mit sich bringen, dass sie die Haare fixieren und die natürliche Beweglichkeit der Frisur einschränken. Eine andere Lösung ist die Verwendung von quaternisierten Pflegestoffen, z.B. kationischen Tensiden oder kationischen Polymeren, die sich am Haar anlagern und zu einer Verminderung der elektrostatischen Aufladung führen. Übliche haarkonditionierende Präparate wie Rinse-off Kuren oder Leave-on Treatments sind in der Regel auf der Basis von wässrigen Emulsionen formuliert. Wesentliche Inhaltsstoffe sind kationaktive Substanzen wie z.B. kationische Tenside, hydrophobe Substanzen wie z.B. Fettalkohole und andere Ölkomponenten, Emulgatoren, sowie weitere spezifische Wirk- und Duftstoffe. Die wichtigsten Bestandteile sind dabei kationische Tenside, Fettalkohole und Emulgatoren. Einen Überblick über den prinzipiellen Aufbau von Kurspülungen und Haarkuren gibt Schrader, 'Grundlagen und Rezepturen der Kosmetika', 2. Auflage, 1989, Seiten 728 bis 737. Hauptaufgaben der Konditioniermittel sind die Verbesserung der Frisierbarkeit, der Kämmbarkeit, des Glanzes und des Griffs des behandelten Haares. Die Anlagerung der kationischen Pflegekomponenten an das Haar führt aber neben den erwünschten Wirkungen in der Regel wegen der relativ hohen Menge an benötigter Pflegekomponente auch zu einer Beschwerung und Belastung des Haares. Eine Belastung führt aber gerade bei feinem Haar zu negativen Frisurergebnissen. Die behandelten Haare fühlen sich schwerer und belasteter an, was nicht immer erwünscht ist.

Es bestand daher die Aufgabe, ein Mittel zur Verfügung zu stellen, welches die typischen Pflegeeigenschaften aufweist, insbesondere den fly-away-Effekt verringert und gleichzeitig dem Haar möglichst einen weniger schweren und belasteten Eindruck verleiht wie nach einer Behandlung mit einem herkömmlichen Kurmittel und ohne dass die Frisur in ihrer Beweglichkeit eingeschränkt wird wie nach einer Behandlung mit einem herkömmlichen Stylingmittel.

Es wurde nun gefunden, dass die Aufgabe gelöst wird durch die Verwendung von elektrisch leitfähigen Polymeren. Gegenstand der Erfindung ist ein kosmetisches Mittel mit einem Gehalt an mindestens einem elektrisch leitfähigen Polymer in einer geeigneten kosmetischen Grundlage.

Um den oben genannten fly-away-Effekt ohne die störenden Nebeneffekte durch herkömmliche Styling- oder Pflegekomponenten zu beseitigen und die statische Aufladung abzubauen, können neuartige, intrinsisch leitfähige Polymere eingesetzt werden. Durch Applikation von Haarbehandlungsmitteln, die diese Polymere enthalten, kann der Fly-Away-Effekt vermieden werden. Diese Polymere sind aus anderen Anwendungsgebieten an sich bekannt. Sie werden zur Behandlung von Oberflächen eingesetzt, um statische Aufladung z.B. von elektronischen Bauteilen oder fotographischen Filmen zu verhindern. Als Dispersion sind sie durch übliche Auftragverfahren wie Spritzen, Drucken, oder Streichen u.ä. verarbeitbar. Es wurde nun gefunden, dass sie sich auch in Haarbehandlungsmittel problemlos einarbeiten lassen. Bei behandelten Haaren ist der fly-away-Effekt deutlich reduziert. Es sind nur sehr geringe Einsatzmengen erforderlich und die Haare fühlen sich leichter und unbelasteter an als bei Behandlung mit herkömmlichen Haarpflegeprodukten.

Intrinsisch leitfähige Polymere sind in der Regel ethylenisch ungesättigt und konjugiert, wodurch ein leichter Ladungstransport im Polymermolekül möglich ist. Derartige Polymere werden auch als organische Metalle bezeichnet. Sie weisen eine Leitfähigkeit von mindestens 10⁻⁵, vorzugsweise von mindestens 10⁻², besonders bevorzugt von mindestens 1 Siemens/cm auf. Geeignete intrinsisch leitfähige Polymere sind beispielsweise ausgewählt aus Polymeren auf Basis von Polyanilin, Polyanisidin, Polydiphenylamin, Polyacetylen, Polythiophen, Polythiopren, Polythienylenvinylen, Bithiophen, Polypyrrol und Polycroconain und deren Derivaten. Derartige Polymere werden häufig mittels Dotierung elektrisch leitfähig gemacht. Dies kann chemisch oder elektrochemisch erfolgen. Durch Behandlung mit Oxidationsmitteln wie Jod, Natriumperoxydisulfat oder Brom oder einer starken Säure werden geeignete Polymere teilweise oxidiert und dadurch elektrisch leitend. Andere Polymere können durch teilweise Reduktion mit Reduktionsmitteln elektrisch leitfähig gemacht werden. Diese Verfahren sind allgemein bekannt. Die Herstellung von intrinsisch leitfähigem Polyanilin und Polypyrrol ist beispielsweise in der EP 0 539 123 beschrieben. Geeignete Polymere sind z.B. Polyradikalkationen. Für eine erhöhte Stabilität der Formulierungen empfiehlt es sich, dass die Polyradikalkationen in Kombination mit polymeren anionischen Verbindungen (Polyanionen) eingesetzt werden und die Zusammensetzungen keine weiteren kationischen Substanzen enthalten, deren Gegenionen um die Polyanionen konkurrieren und zu Ausfällungen führen.

Bevorzugte leitfähige Polymere sind leitfähige Polythiophene, insbesondere leitfähige Polyalkylendioxythiophene. Die Herstellung ist beispielsweise beschrieben in DE 41 18 704 und EP 0 339 340. Ein bevorzugtes leitfähiges Polymer ist 3,4-Polyethylendioxythiophen. Ein geeignetes Handelsprodukt ist Baytron® P von Bayer, eine wässrige Dispersion mit 0,5 Gew.% 3,4-Polyethylendioxythiophen (DEPT) und 0,8 Gew.% Polystyrolsulfonat (PSS). Weitere bevorzugte intrinsisch leitfähige Polymere sind leitfähige Polyaniline, z.B. Versicon® (Allied Signal), ein Polyanilin mit einer Leitfähigkeit von 2-4 S/cm oder Ormecon® (Zipperling Kessler & Co).

Die Einsatzmenge an intrinsisch leitfähigem Polymer kann bei 0,001 bis 5 Gew., vorzugsweise 0,01 bis 1 Gew.%, besonders bevorzugt von 0,02 bis 0,5 Gew.%, bezogen auf die Gesamtzusammensetzung des kosmetischen Mittels liegen.

Das erfindungsgemäße Mittel wird bevorzugt in einem wässrigen oder in einem wässrig-alkoholischen Milieu konfektioniert. Als Alkohole können insbesondere die für kosmetische Zwecke üblicherweise verwendeten niederen Alkohole mit 1 bis 4 C-Atomen wie z.B. Ethanol und Isopropanol enthalten sein. Der Wassergehalt beträgt vorzugsweise von 40 bis 95, besonders bevorzugt von 60 bis 90 Gew.%. Der Alkoholgehalt beträgt vorzugsweise von 1 bis 30, besonders bevorzugt von 5 bis 20 Gew.%. Weitere, besonders bevorzugte wasserlösliche Lösungs- bzw. Feuchthaltemittel sind mehrwertige Alkohole, insbesondere solche mit 2 bis 4 C-Atomen wie z.B. Glycerin, Ethylenglykol oder Propylenglykol in einer Menge von 0,1 bis 10 Gew.%, vorzugsweise von 0,5 bis 5 Gew.%.

Zur weiteren Fixierung des elektrisch leitfähigen Polymers auf dem Haar kann das erfindungsgemäße Mittel zusätzlich mindestens ein weiteres, nicht leitendes, filmbildendes, synthetisches oder natürliches Polymer enthalten. Dieses zusätzliche Polymer hat vorzugsweise nichtionischen, anionischen oder amphoteren Charakter. Kationische Polymere können zu Unverträglichkeiten mit Baytron P führen. Das zusätzliche Polymer wird bevorzugt in einer Menge von 0,5 bis 10 Gew. % eingesetzt. Unter filmbildenden Polymeren werden solche Polymere verstanden, welche in 0,1 bis 5 %iger wässriger, alkoholischer oder wässrigalkoholischer Lösung angewandt in der Lage sind, auf dem Haar einen Polymerfilm zu bilden.

Das erfindungsgemäße Mittel kann darüber hinaus die für Haarbehandlungsmittel üblichen Zusatzbestandteile enthalten, z.B. Parfümöle in einer Menge von vorzugsweise 0,01 bis 5 Gew. %; Netzmittel oder Emulgatoren in einer Menge von vorzugsweise 0,01 bis 10 Gew. %; Feuchthaltemittel; Konservierungsmittel, bakterizide und fungizide Wirkstoffe wie z.B. 2,4,4-Trichlor-2-hydroxydiphenylether, Parabene oder Methylchlorisothiazolinon, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate in einer Menge von 0,1 bis 5 Gew.%; Lichtschutzmittel, Antioxidantien, Radikalfänger, Antischuppenwirkstoffe, Fettalkohole, Glanzgeber, Vitamine und rückfettende Agenzien in einer Menge von 0,01 bis 10 Gew.%.

Das erfindungsgemäße Mittel kann in einem pH-Bereich von 2,0 bis 9,5 vorliegen. Besonders bevorzugt sind schwach saure pH-Werte im Bereich zwischen 4,5 und kleiner 7, besonders bevorzugt von 5,5 bis 6,5. Liegt das erfindungsgemäße Mittel im sauren Bereich vor, so kann es organische oder anorganische Säuren enthalten wie z.B. Ameisensäure, Weinsäure, Äpfelsäure, Maleinsäure, Fumarsäure, Glyoxylsäure, Pyrrolidoncarbonsäure, Zitronensäure, Milchsäure, Schwefelsäure, Essigsäure, Salzsäure, Phosphorsäure u.a..

Das Mittel kann in Form einer Lotion, eines Treibmittel enthaltenden Aerosol-Sprays, eines Non-Aerosol-Sprays, eines Treibmittel enthaltenden Aerosol-Schaums, eines Non-Aerosol-Schaums, eines Gels, einer Creme, einer O/W-Emulsion, einer W/O-Emulsion oder eines Shampoos vorliegen, wobei das elektrisch leitfähige Polymer gelöst oder dispergiert ist. Liegt das Mittel in niedrigviskoser Form vor, so kann es zur Erreichung einer besonders guten Verteilbarkeit auf dem Haar auch versprüht werden. Das erfindungsgemäße Haarbehandlungsmittel liegt dann in Kombination mit einer geeigneten mechanisch betriebenen Sprühvorrichtung vor. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Eine bevorzugte Ausführungsform ist ein Haar- und Körperreinigungsmittel, in welchem das elektrisch leitfähige Polymer zusammen mit mindestens einem waschaktiven Tensid in einer wässrigen Basis enthalten ist. Als waschaktive Tenside können prinzipiell alle zu diesem Zweck üblicherweise in Shampoos oder Duschgelen eingesetzten Tenside verwendet werden. Die Tenside können einzeln oder in Gemischen vorliegen und sind in einer Menge von vorzugsweise 1 bis 50 Gew.%, besonders bevorzugt von 1 bis 30 Gew.% enthalten. Als waschaktive Tenside sind insbesondere nicht-ionische, amphotere, zwitterionische und anionische Tenside geeignet.

Geeignete anionische Tenside sind z.B. ausgewählt aus Alkali- oder Erdalkalisalzen der C10- bis C18-Alkylsulfate, der C10- bis C18-Alkylsulfonate, der C10-bis C18-Alkylbenzolsulfonate, der C10- bis C18-Xylolsulfonate und der mit 1 bis 10 Ethylenoxideinheiten ethoxylierten C10- bis C18-Alkylethersulfate, alpha-Olefinsulfonaten, den ethoxylierten Sulfobernsteinsäurehalbestern der allgemeinen Formel R-(OCH₂CH₂)ₘ-O₂C-CH₂-CH(SO₃M)-COOM
wobei R einen C10- bis C18-Alkylrest bedeutet, M ein Alkali- oder Erdalkalikation darstellt und m eine Zahl von 1 bis 10 bedeutet, insbesondere Sulfosuccinaten wie Disodium Laureth-3 Sulfosuccinat, Disodium PEG-5 Laurylcitrat Sulfosuccinat, Disodium Ricinolamido MEA-Sulfosuccinat oder Disodium Laurylamido MEA-Sulfosuccinat und den Alkylethercarboxylaten der allgemeinen Formel R-(OCH₂CH₂)ₙ-OCH₂-COOM, wobei R einen C10- bis C18-Alkylrest, M ein Alkalioder Erdalkalikation und n eine Zahl von 1 bis 20 bedeutet, insbesondere Ethercarboxylate wie z.B. Sodium Laureth-6 Carboxylat oder Sodium Laureth-11 Carboxylat. In den Haarreinigungsmitteln ist vorzugsweise mindestens einAlkali und Erdalkalisalz der mit 1 bis 10 Ethylenoxideinheiten ethoxylierten C10- bis C18-Alkylethersulfate enthalten.

Geeignete nicht-ionische Tenside sind z.B. alkoxylierte Fettalkohole mit einem hohen Alkoxylierungsgrad, z.B. von 11 bis 50 sowie alkoxylierte Fettsäureester, alkoxylierte Partialglyceride von verzweigten oder unverzweigten, gesättigten oder ungesättigten C6- bis C20-Fettsäuren und einem Ethoxylierungsgrad von 11 bis 400 wie z.B. Polyethylenglykol(200)glycerylpalmitat, alkoxylierte Polyolester wie z.B. ethoxylierte Zuckerester, beispielsweise Polyethylenglykol(120)methylglucosedioleat und Alkylpolyglucoside wie z.B. Coco-Glucoside, Lauryl-Glucoside oder Decyl-Glucoside. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Unter den Fettsäureesterethoxylaten sind vor allem Diglyceridethoxylate zu nennen wie das mit 25 Ethylenoxid-Einheiten ethoxylierte Rizinusöl mit dem INCI-Namen PEG-25 Hydrogenated Castor Oil, das mit 35 Ethylenoxid-Einheiten ethoxylierte Rizinusöl mit dem INCI-Namen PEG-35 Castor Oil, das mit 40 Ethylenoxid-Einheiten ethoxylierte, hydrierte Rizinusöl mit dem INCI-Namen PEG-40 Hydrogenated Castor Oil. Weiterhin können die als nicht-ionische Tenside bekannten ethoxylierten Fettsäurezuckerester, insbesondere der ethoxylierte Sorbitanfettsäureester, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden, für die erfindungsgemäße kosmetische Zubereitung eingesetzt werden.

Geeignete amphotere Tenside sind beispielsweise Betaine wie Cocamidopropylbetain oder Laurylbetain, Sulfobetaine wie z.B. Cocamidopropyl Hydroxysultaine, Glycinate wie z.B. Cocoamphoglycinat (INCl-Bezeichnung: Sodium Cocoamphoacetate) und -diglycinat sowie Propionate wie z.B. Cocoamphopropionat.

Eine weitere bevorzugte Ausführungsform sind Haarkonditioniermittel und zwar sowohl auszuspülende Haarkurmittel (Rinses, Treatments) als auch im Haar verbleibende Produkte (Leave-in Produkte). Diese Haarkurmittel sind in der Regel auf Basis einer O/W-Emulsion hergestellt und enthalten neben dem intrinsisch leitfähigen Polymer Wasser, mindestens einen hydrophoben Stoff (insbesondere mindestens einen Fettalkohol) und einen geigneten Emulgator.

Der Anteil an hydrophoben Stoffen richtet sich nach den Anforderungen des herzustellenden Endproduktes. Er kann für Haarkuren beispielsweise von 5 bis 30 Gew.% oder für Haarcremes auch bis zu ca. 50 Gew.% betragen. Die hydrophile Phase macht dann dementsprechend von 50 bis zu 95 Gew.% aus. Der hydrophobe Stoff in der Ölphase der emulsionsförmigen Haarkur ist ausgewählt aus wasserunlöslichen Wachs-, Fett- oder Ölstoffen. Diese können bei Raumtemperatur flüssig oder fest vorliegen. Geeignet sind Wachse oder wachsartige Stoffe, z.B. natürliche, nachwachsende Wachse (Insekten-, Tier- und Pflanzenwachse), fossile Wachse (Erdöl-, Braunkohlen-, Torfwachse oder Ozokerite), synthetische Wachse (Fischer-Tropsch-, Polyethylen- oder Amidwachse), höherschmelzende Paraffine, Ester, Fette, langkettige Carbonsäuren oder langkettige (C10- bis C22-) Alkohole. Geeignet sind auch Öle oder ölartige Stoffe, z.B. natürlich vorkommende, nachwachsende Öle (pflanzliche und tierische fette Öle), synthetische Öle, Silikonöle, Mineralöle, etherische Öle, wasserunlösliche, verzweigte oder lineare aliphatische Kohlenwasserstoffe, lineare oder verzweigte Alkohole, insbesondere flüssige Fettalkohole sowie langkettige Ether oder Ester, wobei die genannten Substanzen vorzugsweise mindestens 8 C-Atome aufweisen. Geeignete Kohlenwasserstoffe sind z.B. flüssige Paraffine, Squalan oder Squalene. Weiterhin geeignet sind Ester von drei- und mehrwertigen Alkoholen, insbesondere pflanzlichen Triglyceriden wie z.B. Olivenöl, Mandelöl, Erdnußöl, Sonnenblumenöl sowie synthetische Triglyceride wie z.B. C8-C10-Trifettsäureglycerinester oder auch Jojobaöl. Weiterhin als hydrophobe Substanz geeignet sind Mono- oder Diester der Formeln R¹-COOR², R¹-COO-R³-OOCR¹ und R²OOC-R³-COOR², wobei R¹ für eine C8- bis C22-Alkylgruppe, R² für eine C3- bis C22-Alkylgruppe und R³ für eine C2- bis C16-Alkylengruppe steht. Geeignet sind auch natürlich vorkommende Monoester- bzw. Wachsestergemische, wie sie z.B. in Jojobaöl oder Spermöl vorliegen und verzweigte primäre Alkohole, wie sie unter der Bezeichnung Guerbetalkohole bekannt sind. Als hydrophobe Substanzen sind ausserdem Stoffe geeignet, die üblicherweise als Trübungsmittel in kosmetischen Mitteln eingesetzt werden, insbesondere solche der Formel R¹-COO-(CHR⁴CHR⁵O)ₙ-COR⁶, wobei R¹ für eine C8- bis C22-Alkylgruppe, R⁴ und R⁵ für Wasserstoff oder Methyl und R⁶ für Wasserstoff oder für R¹ steht und n eine Zahl zwischen 1 und 12, vorzugsweise 1, 2, 3 oder 4 bedeutet. Bevorzugt sind Glykoldifettsäureester und Polyethylenglykoldifettsäureester, welche bei Raumtemperatur in fester Form vorliegen.

Der in der emulsionsförmigen Haarkur enthaltene Emulgator kann in Mengen von 0,5 bis 30 Gew.% der fertigen Zusammensetzung vorliegen. Als Emulgatoren sind insbesondere nicht-ionische und anionische Emulgatoren geeignet. Geeignete Emulgatoren sind beispielsweise die im 'International Cosmetic Ingredient Dictionary and Handbook', 7.Auflage, Band 2 im Abschnitt 'Surfactants', insbesondere im Unterabschnitt 'Surfactants - Emulsifying Agents' aufgeführten Emulgatoren. Geeignete nichtionische Emulgatoren sind die oben genannten nichtionischen Tenside, vorzugsweise oxethylierte Fettalkohole, oxethylierte Nonylphenole, Alkylpolyglycoside, Fettsäuremono- und -diglyceride, ethoxyliertes und hydriertes oder nicht hydriertes Rizinusöl, Fettsäurealkanolamide und oxethylierte Fettsäureester. Geeignete anionische Emulgatoren können aus den oben genannten anionischen Tensiden ausgewählt werden.

Das erfindungsgemäße Konditioniermittel wird angewendet, indem eine für den gewünschten Konditioniereffekt ausreichende Menge in oder auf dem trockenen Haar oder nach der Haarwäsche in oder auf dem nassen oder feuchten Haar verteilt wird. Die anzuwendende Menge hängt von der Haarfülle ab und beträgt typischerweise 1 bis 25 g. Bei der bevorzugten Verwendung als Rinse-Produkt wird nach einer ausreichenden Einwirkzeit von beispielsweise 1 bis 15 Minuten das Haar ausgespült. Anschließend wird das Haar gegebenenfalls durchgekämmt oder zur Frisur geformt und getrocknet. Bei einer Verwendung als Leavein-Produkt wird das Haar nach Aufbringen des Mittels nicht ausgespült.

Die nachfolgenden Beispiele sollen den Gegenstand der Erfindung näher erläutern.

| **Beispiel 1:** Shampoos mit intrinsisch leitfähigem 3,4-Polyethylendioxythiophen | | | | | | | |
|---|---|---|---|---|---|---|---|
| **Rohstoffname** | **A** | **B** | **C** | **D** | **E** | **F** | **G** |
| Texapon® N28 Benz ¹ | 35,0 % | 35,0 % | 35,0 % | 35,0 % | 35,0 % | 35,0 % | 35,0 % |
| Baytron® P ² | - | 1,0% | 3,0% | 5,0% | 10,0% | 15,0% | 20,0% |
| Natriumchlorid | 4,0% | 4,0% | 4,0% | 4,0% | 4,0% | 4,0% | 4,0% |
| Dest. Wasser | ---------------------- ad 100 % ---------------------------------- | | | | | | |

| | | | | | | | |
|---|---|---|---|---|---|---|---|
| ¹ Sodium Laureth Sulfate, 28%ig in Wasser, konserviert mit Benzoesäure | | | | | | | |
| ² wässrige Dispersion von 0,5 Gew.% 3,4-Polyethylendioxythiophen und 1,3 Gew.% Polystyrolsulfonat | | | | | | | |

Zur Überprüfung des Fly-Away-Effektes werden Perückenköpfe im Halbseitenversuch gewaschen. Eine Kopfhälfte wird mit 6 ml Shampoo gewaschen (ca. 1 Min.) und anschließend mit warmem Wasser wieder ausgespült (ca. 5 Min.). Der Perückenkopf wird zuerst mit einem Handtuch vorgetrocknet und danach mit einem Handhaartrockner. Vor der Überprüfung des Fly-Away-Effektes wird der Perückenkopf zwei Tage im Klimaraum bei 20°C und 65% relativer Luftfeuchtigkeit konditioniert. Die Haare werden mit einer Nylonbürste gekämmt. Die Verhinderung des Fly-Away-Effektes ist ab einem Zusatz von 5 % Baytron P (0,025 Gew.% 3,4-Polyethylendioxythiophen) deutlich erkennbar.

| **Beispiel 2:** Haarkur mit intrinsisch leitfähigem 3,4-Polyethylendioxythiophen | | | |
|---|---|---|---|
| Rohstoff | Muster A | Muster B | Muster C |
| Cetearylalkohol | 1,5% | 1,5% | 1,5% |
| PPG-12-PEG-65 Lanolin Oil | 1,2% | 1,2% | 1,2% |
| Vaseline | 1,5% | 1,5% | 1,5% |
| Natriumcetearylsulfat | 0,6 % | 0,6 % | 0,6 % |
| Sorbitol | 3,5 % | 3,5 % | 3,5 % |
| Zitronensäure | 0,2 % | 0,2 % | 0,2 % |
| Ethanol | 12,0% | 12,0 % | 12,0 % |
| Baytron P | - | 5,0 % | 10,0% |
| Wasser dest. | Ad 100% | Ad 100% | Ad 100% |

Zur Überprüfung des Fly-Away-Effektes wird ein Perückenkopf im Halbseitenversuch behandelt. Eine Kopfhälfte wird mit 10 ml Haarkur A behandelt (Einwirkzeit ca. 2 Min.) und anschließend mit warmem Wasser wieder ausgespült (ca. 5 Min.). Die andere Kopfhälfte wird mit Haarkur B bzw. C behandelt. Der Perückenkopf wird zuerst mit einem Handtuch vorgetrocknet und danach mit einem Handhaartrockner. Vor der Überprüfung des Fly-Away-Effektes wird der Perückenkopf zwei Tage im Klimaraum bei 20°C und 30% Luftfeuchtigkeit konditioniert. Die Haare werden mit einer Nylonbürste gekämmt. Der Zusatz von Baytron P bewirkt einen positiven Effekt, die Haare haben eine geringere statische Aufladung.

## Patentansprüche

1. Kosmetisches Mittel mit einem Gehalt an mindestens einem elektrisch leitfähigen Polymer in einer geeigneten kosmetischen Grundlage.

2. Mittel nach Anspruch 1, **dadurch gekennzeichnet, dass** die Leitfähigkeit des Polymers mindestens 10⁻⁵ S/cm beträgt.

3. Mittel nach einem der vorhergehenden Ansprüche, **dadurch gekennzeichnet, dass** das elektrisch leitfähige Polymer ausgewählt ist aus elektrisch leitfähigen Polymeren auf Basis von Polyanilin, Polyanisidin, Polyacetylen, Polydiphenylamin, Polyacetylen, Polythiopren, Polythienylenvinylen, Polythiophen, Polycroconain und Polypyrrol.

4. Mittel nach Anspruch 1, **dadurch gekennzeichnet, dass** das elektrisch leitfähige Polymer ein Polyradikalkation ist.

5. Mittel nach Anspruch 4, **dadurch gekennzeichnet, dass** das Polyradikalkation in Kombination mit einem Polyanion vorliegt.

6. Mittel nach Anspruch 1, **dadurch gekennzeichnet, dass** das Polymer elektrisch leitfähiges 3,4-Polyethylendioxythiophen oder elektrisch leitfähiges Polyanilin ist.

7. Mittel nach Anspruch 6, **dadurch gekennzeichnet dass** das elektrisch leitfähige 3,4-Polyethylendioxythiophen in Kombination mit Polystyrolsulfonat vorliegt.

8. Mittel nach einem der vorhergehenden Ansprüche, **dadurch gekennzeichnet, dass** das elektrisch leitfähige Polymer in einer Menge von 0,001 bis 5 Gew.% enthalten ist.

9. Mittel nach einem der vorhergehenden Ansprüche, **dadurch gekennzeichnet, dass** es in Form einer Lotion, eines Aerosol-Sprays, eines Non-Aerosol-Sprays, eines Aerosol-Schaums, eines Non-Aerosol-Schaums, eines Gels, einer Creme, einer O/W-Emulsion, einer W/O-Emulsion oder eines Shampoos vorliegt, wobei das elektrisch leitfähige Polymer gelöst oder dispergiert ist.

10. Haar- oder Körperreinigungsmittel, enthaltend mindestens ein elektrisch leitfähiges Polymer und mindestens ein waschaktives Tensid in einer wässrigen Grundlage.

11. Haarkonditioniermittel enthaltend mindestens ein elektrisch leitfähiges Polymer in einer Grundlage auf Basis einer O/W-Emulsion.

12. Verwendung eines elektrisch leitfähigen Polymers zur Behandlung von Haaren.

13. Verwendung eines elektrisch leitfähigen Polymers zur Reduzierung des Flyaway-Effektes von Haaren.
